# EUROPEAN PATENT APPLICATION

(11) **EP 4 847 128 A1**
(43) Date of publication of application: **02.12.2026**
(21) Application number: 25745323.3
(22) Date of filing: 22.01.2025
(51) Int. Cl.: B01J 23/72, B01J 37/00, B01J 37/08, B01J 21/08, B01J 35/37, B01J 35/61, B01J 35/40, B01J 37/03, C07C 29/141, C07C 31/20

(54) **COPPER SILICATE-BASED CATALYST AND METHOD FOR MANUFACTURING SAME**

(30) Priority: 23.01.2024 KR 20240009938
(71) Applicant: LG CHEM, LTD., Seoul 07336 (KR)
(72) Inventor: SUH, Myungji, Daejeon 34122 (KR); EOM, Sungshik, Daejeon 34122 (KR)
(74) Representative: Goddar, Heinz J.
(86) International application number: PCT/KR2025/001241
(87) International publication number: WO 2025/159498

(57) **Abstract**

A copper silicate-based catalyst according to an exemplary embodiment of the present application is a copper silicate-based catalyst containing copper and silica, wherein the copper silicate-based catalyst has a crushing strength of 40 N or higher, the copper silicate-based catalyst has a Cu surface area of 3 m²/g to 10 m²/g upon N₂O pulse chemisorption analysis, and the copper silicate-based catalyst has a Si elution ratio of 5 wt% or lower after an accelerated test according to Method 1.

## Description

### [Technical Field]

This application claims the benefit of Korean Patent Application No. 10-2024-0009938 filed in the Korean Intellectual Property Office on January 23, 2024, the entire contents of which are incorporated herein by reference.

This application relates to a copper silicate-based catalyst and a method for preparing the same.

### [Background Art]

Neopentyl glycol (NPG) is a white crystalline substance with a melting point of 130 °C or higher. It is used as an important intermediate for various synthetic resins and is also widely used industrially as a raw material for various plastic powder paints, synthetic lubricants, plasticizers, surfactants, fiber processing agents, etc.

The NPG is generally prepared by preparing hydroxypivaldehyde (HPA) through an aldol condensation reaction of isobutyraldehyde and formaldehyde, and then reacting the HPA with hydrogen in the presence of a catalyst.

Conventionally, the HPA has been subjected to hydrogenation reaction using a slurry-type Ni-based catalyst. In this case, crude NPG, which is a hydrogenation reaction product, contains 2,2,4-trimethyl-1,3-pentanediol (TMPD), hydroxypivalic acid NPG ester (HPNE), etc. Since TMPD and HPNE have boiling points very similar to that of NPG, they cannot be separated by simple distillation. Since HPNE is unstable when distilling the reaction mixture and leads to a decrease in the yield of NPG, it is commercially converted to NPG by saponification reaction by adding sodium hydroxide. However, since sodium salts of HPA or other organic acids produced by the saponification reaction promote the decomposition reaction of NPG at high temperatures of 140 °C or higher, the distillation process is constrained. Furthermore, it is impossible to remove TMPD which has not been converted to a non-volatile sodium salt during the saponification reaction.

Therefore, efforts for producing NPG at a high yield in an economical way are being made continuously in the art.

### [Disclosure]

### [Technical Problem]

The present application provides a copper silicate-based catalyst and a method for preparing the same.

### [Technical Solution]

An exemplary embodiment of the present application provides a copper silicate-based catalyst containing copper and silica, wherein
the copper silicate-based catalyst has a crushing strength of 40 N or higher,
the copper silicate-based catalyst has a Cu surface area of 3 m²/g to 10 m²/g upon N₂O pulse chemisorption analysis, and
the copper silicate-based catalyst has a Si elution ratio of 5 wt% or lower after an accelerated test according to Method 1:

### [Method 1]

After immersing the copper silicate-based catalyst in a hydroxypivaldehyde (HPA) solution and conducting reaction at 180 °C for 6 hours under hydrogen atmosphere, the elution ratio of Si is calculated according to Equation 1. [Equation 1] 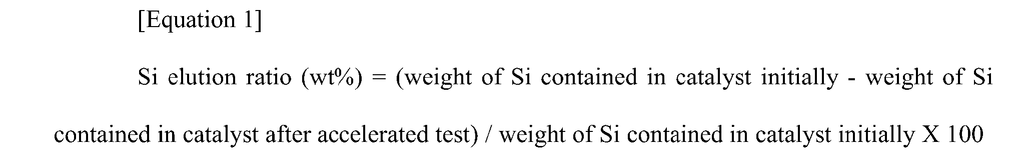 Si elution ratio (wt%) = (weight of Si contained in catalyst initially - weight of Si contained in catalyst after accelerated test) / weight of Si contained in catalyst initially X 100

In addition, another exemplary embodiment of the present application provides a method for preparing a copper silicate-based catalyst, which comprises:
a step of preparing a coprecipitated slurry by adding a silica sol and an alkaline precipitant to an aqueous solution containing a copper precursor;
a step of aging the coprecipitated slurry at 80 °C to 90 °C for 3 hours or longer, and then preparing a dried product by filtering and drying the same;
a step of mixing the dried product with an inorganic binder and an organic compound, and then preparing a catalyst precursor by molding the same; and
a step of drying and sintering the catalyst precursor,
wherein temperature is maintained at 15 °C to 35 °C during the addition of the alkaline precipitant.

In addition, another exemplary embodiment of the present application provides a method for preparing neopentyl glycol, which comprises a step of conducting hydrogenation reaction by adding a hydroxypivaldehyde (HPA) solution and hydrogen to a hydrogenation reactor,
wherein the hydrogenation reactor contains the copper silicate-based catalyst.

### [Advantageous Effects]

According to an exemplary embodiment of the present application, a copper silicate-based catalyst with improved stability can be prepared. In particular, since the copper silicate-based catalyst according to an exemplary embodiment of the present application has a Si elution ratio of 5 wt% or lower after an accelerated test, the decline in the strength, reactivity, etc. of the catalyst may be prevented even under the high-temperature, high-pressure reaction condition for preparation of neopentyl glycol with a high yield.

In addition, according to an exemplary embodiment of the present application, the Cu surface area of the catalyst can be optimized by controlling the aging condition of a coprecipitated slurry, the addition condition of an alkaline precipitant, and the drying condition of a catalyst precursor.

Accordingly, when the copper silicate-based catalyst according to an exemplary embodiment of the present application is used to prepare neopentyl glycol, the preparation process can be maintained stably and, therefore, economical efficiency can be enhanced.

### [Brief Description of Drawings]

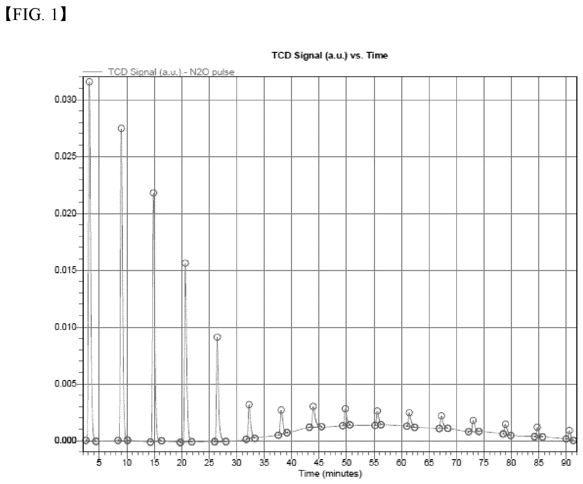

FIG. 1 shows the result of N₂O chemisorption analysis for measuring the Cu surface area of a copper silicate-based catalyst of Example 1 according to the present application.

### [Best Mode]

Hereinafter, the present specification is described in more detail.

In the present specification, when a member is described to be located "on" another member, it comprises not only the case where the two members are in contact with each other but also the case where another member is present between the two members.

In the present specification, when a part is described to "comprise" a certain component, it means that it may comprise other components and does not exclude other components, unless the context specifically states otherwise.

As stated above, efforts for producing NPG at a high yield in an economical way are being made continuously in the art.

In particular, neopentyl glycol is prepared by hydrogenation of HPA (hydroxypivaldehyde) in the presence of a catalyst under high-temperature (160 °C or higher) and high-pressure (35 bar or higher) conditions. Since the strength and reactivity of the catalyst may decline under the high-temperature, high-pressure reaction conditions due to the elution of catalyst components, the hydrogenation process may not be operated stably.

Therefore, the present application is directed to providing a copper silicate-based catalyst with superior stability, which can be applied to the preparation process of neopentyl glycol, and a method for preparing the same.

The copper silicate-based catalyst according to an exemplary embodiment of the present application contains copper and silica, wherein the copper silicate-based catalyst has a crushing strength of 40 N or higher, the copper silicate-based catalyst has a Cu surface area of 3 m²/g to 10 m²/g upon N₂O pulse chemisorption analysis, and the copper silicate-based catalyst has a Si elution ratio of 5 wt% or lower after an accelerated test according to Method 1.

### [Method 1]

After immersing the copper silicate-based catalyst in a hydroxypivaldehyde (HPA) solution and conducting reaction at 180 °C for 6 hours under hydrogen atmosphere, the elution ratio of Si is calculated according to Equation 1. [Equation 1] 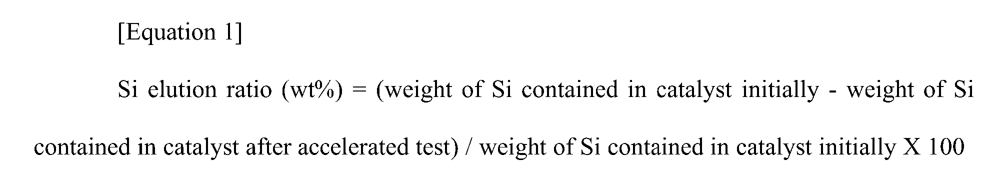 Si elution ratio (wt%) = (weight of Si contained in catalyst initially - weight of Si contained in catalyst after accelerated test) / weight of Si contained in catalyst initially X 100

The copper silicate-based catalyst may have a Si elution ratio of 5 wt% or lower, 3 wt% or lower, or 0, after an accelerated test according to Method 1. If the Si elution ratio of the copper silicate-based catalyst exceeds 5 wt% after an accelerated test according to Method 1, the strength and reactivity of the catalyst may decline due to elution of Si during a preparation process of neopentyl glycol by hydrogenation of HPA under high-temperature, high-pressure reaction conditions. This may cause problems in stable operation of the hydrogenation process.

In an exemplary embodiment of the present application, the copper silicate-based catalyst may have a crushing strength of 40 N or higher, specifically 42 N to 150 N, or 49 N to 67 N. If the crushing strength of the copper silicate-based catalyst is lower than 40 N, the catalyst may be damaged easily when it is put in a reactor, and a differential pressure may be formed undesirably in the reactor due to damage within a relatively short period.

A specific method for measuring the crushing strength of the copper silicate-based catalyst is described in the examples given below.

In an exemplary embodiment of the present application, the copper silicate-based catalyst may have a crushing strength decline ratio of 10% or lower, 5% or lower, 3% or lower, or 0, after an accelerated test according to Method 2.

### [Method 2]

After immersing the copper silicate-based catalyst in a hydroxypivaldehyde (HPA) solution and conducting reaction at 180 °C for 6 hours under hydrogen atmosphere, the crushing strength decline ratio is calculated according to Equation 2. [Equation 2] 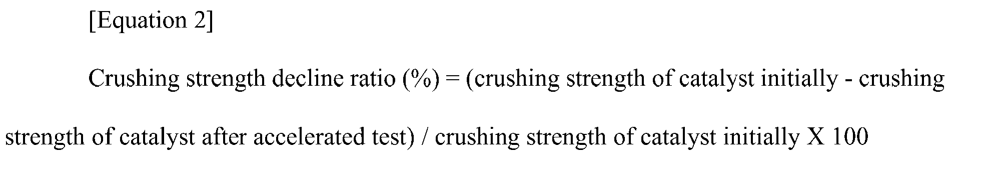 Crushing strength decline ratio (%) = (crushing strength of catalyst initially - crushing strength of catalyst after accelerated test) / crushing strength of catalyst initially X 100

If the crushing strength decline ratio of the copper silicate-based catalyst exceeds 10% after an accelerated test according to Method 2, the strength and reactivity of the catalyst may decline due to elution of Si during a preparation process of neopentyl glycol by hydrogenation of HPA under high-temperature, high-pressure reaction conditions. This may cause problems in stable operation of the hydrogenation process.

In an exemplary embodiment of the present application, the copper silicate-based catalyst may have a Cu surface area of 3 m²/g to 10 m²/g, 4 m²/g to 9 m²/g, or 5 m²/g to 9 m²/g, upon N₂O chemisorption analysis. If the Cu surface area of the copper silicate-based catalyst upon N₂O chemisorption analysis is outside the above ranges, catalytic activity may decline undesirably.

A specific method for measuring the Cu surface area of the copper silicate-based catalyst by N₂O chemisorption analysis is described in the examples given below.

In an exemplary embodiment of the present application, the weight ratio of Cu : Si in the copper silicate-based catalyst may be 30 : 70 to 70 : 30, 30 : 70 to 50 : 50, or 35 : 65 to 50 : 50. The weight ratio of Cu : Si in the copper silicate-based catalyst is an indicator of the content of Cu, which is the active component of the catalyst. If the weight ratio of Cu : Si is outside the above ranges, the activity of the catalyst declines undesirably.

In an exemplary embodiment of the present application, the copper silicate-based catalyst may be a catalyst extrusion-molded into a cylindrical shape with a diameter of 2 mm to 6 mm and a height of 2 mm to 10 mm, or a catalyst extrusion-molded into a cylindrical shape with a diameter of 3 mm to 5 mm and a height of 3 mm to 9 mm. If the diameter of the cylindrical catalyst exceeds 6 mm or its height exceeds 10 mm, catalytic activity may decrease undesirably due to decreased surface area. In addition, if the diameter of the cylindrical catalyst is smaller than 2 mm or its height is smaller than 2 mm, crushing and drifting may occur undesirably under the high-temperature, high-pressure reaction condition for preparation of neopentyl glycol.

The molding of the catalyst is important for physical durability and flow of reactants in the reactor. Although a powder catalyst may be advantageous in terms of catalytic activity because of large surface area due to small particle size, the flow of reactants in a commercial-scale fixed-bed reactor is limited and drifting or differential pressure may occur. For this reason, it is desirable to mold the catalyst by extrusion, compression, coating, etc. The pores formed by the molded catalyst in the fixed-bed reactor provide the effect of reducing differential pressure caused by reactants supplied at a high flow rate for commercial productivity. In particular, since the extrusion-molded catalyst has the advantage of high durability due to high strength, the possibility of drifting or differential pressure caused by wearing, crushing, etc. during long-term use of the catalyst may be decreased.

In an exemplary embodiment of the present application, the copper silicate-based catalyst may be for preparation of neopentyl glycol.

In addition, a method for preparing a copper silicate-based catalyst according to an exemplary embodiment of the present application comprises: a step of preparing a coprecipitated slurry by adding a silica sol and an alkaline precipitant to an aqueous solution containing a copper precursor; a step of aging the coprecipitated slurry at 80 °C to 90 °C for 3 hours or longer, and then filtering the same; a step of preparing a dried product by drying the filtered coprecipitated slurry at 80 °C to 90 °C; a step of mixing the dried product with an inorganic binder comprising a fiber-based binder and graphite; and an organic compound, and then preparing a catalyst precursor by molding the same; and a step of drying and sintering the catalyst precursor, wherein temperature is maintained at 15 °C to 35 °C during the addition of the alkaline precipitant.

The method for preparing a copper silicate-based catalyst according to an exemplary embodiment of the present application comprises a step of preparing a coprecipitated slurry by adding a silica sol and an alkaline precipitant to an aqueous solution containing a copper precursor.

In an exemplary embodiment of the present application, the copper precursor may be Cu(NO₃)₂·3H₂O, Cu(CO₃)₂·Cu(OH)₂, CuCl₂·2H₂O, etc., although not being limited thereto.

In an exemplary embodiment of the present application, the alkaline precipitant may be an alkali metal hydroxide, an alkali metal carbonate, an alkali metal bicarbonate, a mixture thereof, etc. More specifically, the alkaline precipitant may be one or more of NH₄OH, (NH₄)₂CO₃, NH₄HCO₃, CH₄N₂O, NaOH and Na₂CO₃, although not being limited thereto.

In an exemplary embodiment of the present application, temperature is maintained at 15 °C to 35 °C during the addition of the alkaline precipitant. When adding the alkaline precipitant, the temperature increased by heat of neutralization may be lowered to 15 °C to 35 °C using cooling water, etc.

During the addition of the alkaline precipitant, the temperature is maintained as described above to control the heat of neutralization. The initial particle size of the coprecipitated slurry may change depending on the degree of control of the heat of neutralization, and the activity and physical stability of the catalyst may be affected thereby. If the temperature is below 15 °C during the addition of the alkaline precipitant, Si may be eluted easily, and this may weaken the physical stability of the catalyst undesirably. In addition, if the temperature is maintained above 35 °C during the addition of the alkaline precipitant, catalytic activity may decline undesirably due to change in the crystal phase of the coprecipitated slurry.

The method for preparing a copper silicate-based catalyst according to an exemplary embodiment of the present application comprises a step of aging the coprecipitated slurry at 80 °C to 90 °C for 3 hours or longer, and then filtering the same.

The aging may be performed at 80 °C to 90 °C for 3 hours or longer, or at 80 °C to 90 °C for 3 hours to 8 hours.

Bonding between the copper precursor and the silica is formed by the aging. The copper precursor does not remain unreacted when both the aging temperature and time described above are satisfied. If the copper precursor remains unreacted, the activity of the catalyst may decrease undesirably.

After the aging, the coprecipitated slurry may be filtered by a method known in the art without special limitation.

The method for preparing a copper silicate-based catalyst according to an exemplary embodiment of the present application comprises a step of preparing a dried product by drying the filtered coprecipitated slurry at 80 °C to 90 °C. If the drying condition of the filtered coprecipitated slurry is outside the above range, catalytic activity may decline undesirably due to the change in the crystallinity of the catalyst.

The method for preparing a copper silicate-based catalyst according to an exemplary embodiment of the present application comprises a step of mixing the dried product with an inorganic binder containing a fiber-based binder and graphite; and an organic compound, and then preparing a catalyst precursor by molding the same.

In an exemplary embodiment of the present application, the fiber-based binder may comprise one or more of glass fiber, carbon fiber, aramid fiber, alumina fiber, aluminum silicate fiber, silicon carbide fiber and boron fiber.

In an exemplary embodiment of the present application, the content of the inorganic binder may be 0.1 wt% or more and less than 15 wt%, 1 wt% to 10 wt%, 7 wt% to 10 wt%, based on the total weight of the dried product. If the content of the inorganic binder is less than 0.1 wt% based on the total weight of the dried product, physical stability may worsen undesirably under the high-temperature, high-pressure reaction condition due to significantly decreased strength of the catalyst. And, if the content of the inorganic binder is 15 wt% or more, productivity may decrease during the preparation of the catalyst because of poor flowability during the extrusion molding of the catalyst and the strength of the catalyst may also decline because of poor compressibility.

In an exemplary embodiment of the present application, the content of the organic compound may be 0.1 wt% to 50 wt%, 1 wt% to 40 wt%, or 10 wt% to 30 wt%, based on the total weight of the dried product. The organic compound serves as a lubricant during the molding of the catalyst. If the content of the organic compound is less than 0.1 wt% based on the total weight of the dried product, molding may be impossible. And, if it exceeds 50 wt%, the strength or activity of the catalyst may decline undesirably.

In an exemplary embodiment of the present application, the organic compound may comprise one or more of polyvinyl alcohol, isopropyl alcohol, ethanol, polyacrylate, polyethylene glycol, glycerin, starch, dextrin, wax, methyl cellulose, carboxymethyl cellulose, hydroxypropyl methyl cellulose, paraffin, lignosulfonate, stearic acid and palmitic acid.

The method for preparing a copper silicate-based catalyst according to an exemplary embodiment of the present application comprises a step of drying and sintering the catalyst precursor.

In an exemplary embodiment of the present application, the catalyst precursor may be dried at 60 °C to 120 °C for 5 hours to 24 hours, or at 70 °C to 110 °C for 7 hours to 20 hours.

In an exemplary embodiment of the present application, the dried catalyst precursor may be sintered at 350 °C to 600 °C for 5 hours to 24 hours, or at 400 °C to 550 °C for 7 hours to 20 hours.

If the drying condition and sintering condition are outside the above ranges, catalytic activity may decline undesirably due to change in the crystallinity of the catalyst.

In addition, another exemplary embodiment of the present application provides a method for preparing neopentyl glycol, which comprises a step of adding a hydroxypivaldehyde (HPA) solution and hydrogen to a hydrogenation reactor and conducting hydrogenation reaction, wherein the hydrogenation reactor contains the copper silicate-based catalyst.

In an exemplary embodiment of the present application, a method known in the art may be used for the preparation of neopentyl glycol, except for using the copper silicate-based catalyst.

For example, the hydrogenation reactor may be a fixed-bed reactor (FBR) charged with the copper silicate-based catalyst. In this case, it is not necessary to separate the reaction product from the catalyst, the operation is stable and economical because reaction temperature and reaction pressure can be lowered, the replacement of the catalyst is easy, and the investment cost can be reduced greatly because the reactor size can be decreased.

In addition, the hydroxypivaldehyde solution may contain 65 wt% or less of hydroxypivaldehyde, 10 wt% or less of neopentyl glycol, 15 wt% to 35 wt% of an alcohol, and 15 wt% or less of water. In this case, the production of byproducts may be suppressed since the heat of reaction can be minimized without sacrificing reactivity.

The hydrogenation reaction may be performed at 100 °C to 250 °C, 100 °C to 200 °C, or 100 °C to 180 °C.

According to an exemplary embodiment of the present application, a copper silicate-based catalyst with improved stability may be prepared. In particular, since the copper silicate-based catalyst according to an exemplary embodiment of the present application has a Si elution ratio of 5 wt% or lower after an accelerated test, the decline in the strength, reactivity, etc. of the catalyst may be prevented even under the high-temperature, high-pressure reaction condition for preparation of neopentyl glycol with a high yield.

In addition, according to an exemplary embodiment of the present application, the Cu surface area of the catalyst may be optimized by controlling the aging condition of the coprecipitated slurry, the addition condition of the alkaline precipitant, the drying condition of the filtered coprecipitated slurry, and the type of the inorganic binder.

Accordingly, when the copper silicate-based catalyst according to an exemplary embodiment of the present application is used to prepare neopentyl glycol, the preparation process can be maintained stably and, therefore, economical efficiency can be enhanced.

### [Mode for Invention]

Hereinafter, the present application will be described in detail through specific examples. However, the examples according to the present application can be changed into various other forms, and it should not be interpreted that the scope of the present application is limited by the examples described below. The examples of the present application are provided to describe the present application more completely to those having ordinary knowledge in the art.

### <Examples>

### <Example 1>

A copper precursor (Cu(NO₃)₂·3H₂O) aqueous solution was prepared in a double jacket reactor while maintaining temperature at 30 °C, and silica sol was added such that the weight ratio of Cu and Si was Cu : Si = 45 : 55. After adding a NaOH aqueous solution while maintaining temperature at 30 °C, a coprecipitate was prepared by increasing the temperature of the reactor and performing aging at 80 °C for 3 hours. After filtering the coprecipitate and washing with distilled water, a wet cake obtained thereby was dried in an oven at 80 °C oven for 24 hours. The dried product was crushed to obtain a catalyst intermediate powder.

After mixing 10 wt% of an organic compound (8 wt% of isopropyl alcohol and 2 wt% of glycerin), 0.1 wt% of a fiber-based binder (ceramic wool, aluminum silicate) and 6.9 wt% of graphite, based on the total weight of the catalyst intermediate powder, the catalyst intermediate was extrusion-molded into a cylindrical shape with a diameter of 3 mm to 5 mm and a height of 3 mm to 9 mm using an extruder.

Finally, a catalyst for preparation of neopentyl glycol was prepared by sintering the extrusion-molded catalyst intermediate at 550 °C for 8 hours.

### <Example 2>

A catalyst for preparation of neopentyl glycol was prepared in the same manner as in Example 1, except that 20 wt% of an organic compound (20 wt% of isopropyl alcohol and 5 wt% of glycerin), 1 wt% of a fiber-based binder and 9 wt% of graphite were used in the extrusion molding step, instead of 10 wt% of the organic compound, 0.1 wt% of the fiber-based binder and 6.9 wt% of the graphite.

### <Example 3>

A catalyst for preparation of neopentyl glycol was prepared in the same manner as in Example 1, except that 20 wt% of an organic compound (20 wt% of isopropyl alcohol and 5 wt% of glycerin), 3 wt% of a fiber-based binder and 7 wt% of graphite were used in the extrusion molding step, instead of 10 wt% of the organic compound, 0.1 wt% of the fiber-based binder and 6.9 wt% of the graphite.

### <Comparative Example 1>

A catalyst for preparation of neopentyl glycol was prepared in the same manner as in Example 1, except that temperature was controlled to 45 °C during the addition of the NaOH aqueous solution by maintaining the temperature of the double jacket reactor at 45 °C.

### <Comparative Example 2>

A catalyst for preparation of neopentyl glycol was prepared in the same manner as in Example 1, except that aging was performed at 95 °C for 3 hours, rather than at 80 °C for 3 hours.

### <Comparative Example 3>

A catalyst for preparation of neopentyl glycol was prepared in the same manner as in Example 1, except that the wet cake was dried in an oven at 95 °C, rather than in an oven at 80 °C oven.

### <Comparative Example 4>

A catalyst for preparation of neopentyl glycol was prepared in the same manner as in Example 1, except that the fiber-based binder and graphite were not used in the extrusion molding step.

### <Comparative Example 5>

A catalyst for preparation of neopentyl glycol was prepared in the same manner as in Example 1, except that the organic compound was not used in the extrusion molding step.

### <Comparative Example 6>

A catalyst for preparation of neopentyl glycol was prepared in the same manner as in Example 1, except that the fiber-based binder, graphite and organic compound were not used in the extrusion molding step. However, extrusion was impossible because lubrication was not performed smoothly in the extruder during the extrusion molding step. As a result, a catalyst could not be prepared.

### <Test Example>

In the presence of the catalyst prepared in the examples or comparative examples, hydrogenation reaction was performed at 160 °C and 35 bar for 1 hour using an HPA solution consisting of 65 wt% of HPA, 2 wt% of NPG, 25 wt% of 2-ethylhexanol, 5 wt% of H₂O and 3 wt% of a high-boiling-point material, and catalytic activity was measured by measuring the amount of hydrogen consumed. The result is shown in Table 1. The catalytic activity was represented relative to the result of Example 1 as 100.

In addition, the crushing strength of the catalysts of the examples and comparative examples and the average particle size of the coprecipitated slurry were evaluated. The result is shown in Table 1.

In addition, the Cu surface area of the catalysts of the examples and comparative examples was evaluated by N₂O chemisorption analysis, and Si elution ratio and strength decline ratio were evaluated by an accelerated test. The result is shown in Table 2.

FIG. 1 shows the result of N₂O chemisorption analysis for measuring the Cu surface area of the copper silicate-based catalyst of Example 1.

The measurement methods of the evaluation results shown in Tables 1 and 2 are as follows.

### <Crushing strength>

Crushing strength was measured using SHIMPO's FGN-50B. More specifically, after placing the catalyst extrusion-molded to a cylindrical shape on a strength tester holder such that the side surface faces the bottom, the maximum pressure value was measured as the measuring unit compresses the catalyst downward from the top. Measurement was made for 20 catalysts and the result was averaged.

For Example 1, although it was intended to measure the crushing strength of the catalyst intermediate powder without conducting the extrusion molding step, the measurement was impossible with the method using SHIMPO's FGN-50B because the particle size of the powder catalyst was small as about 100 µm. Thus, the crushing strength of the powder catalyst was evaluated as 0.

### <Average particle size of coprecipitated slurry>

The average particle size of the coprecipitated slurry was measured by a wet particle size analysis method.

More specifically, after background measurement using Microtrac's S3500, about 3 drops of the coprecipitated slurry were added under the condition of ultrasonic vibration and average particle size was determined by measuring particle size distribution.

### <Cu surface area>

Cu surface area was measured by N₂O chemisorption analysis.

More specifically, using Micromeritics' AutoChem, about 0.2 g of a catalyst sample was filled in a U-shaped tube and pre-treated at 250 °C for 2 hours under hydrogen atmosphere. Then, after pulse-injecting N₂O at 60 °C using a loop of a predetermined volume, Cu surface area was calculated from the accumulated consumption amount of N₂O and the Cu wt% of the catalyst.

### <Accelerated test>

After immersing the catalyst of the examples or comparative examples in a HPA solution, accelerated test was performed by conducting reaction at 180 °C for 6 hours under hydrogen atmosphere. Then, the solution remaining on the catalyst was removed by washing with acetone and water and then drying at room temperature. The elution ratio of Si was calculated according to Equation 1. In addition, the crushing strength decline ratio of the catalyst was calculated according to Equation 2. [Equation 1] 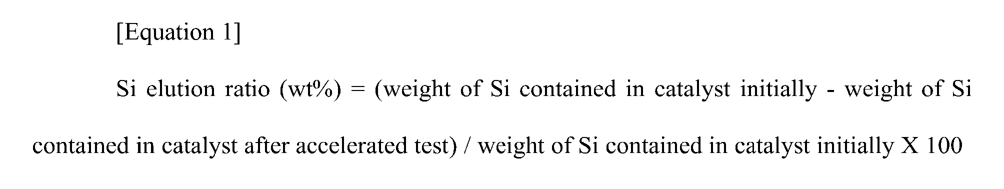 Si elution ratio (wt%) = (weight of Si contained in catalyst initially - weight of Si contained in catalyst after accelerated test) / weight of Si contained in catalyst initially X 100 [Equation 2] 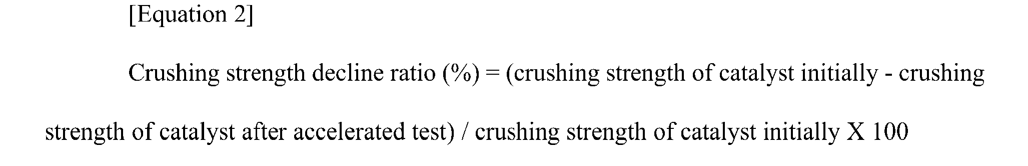 Crushing strength decline ratio (%) = (crushing strength of catalyst initially - crushing strength of catalyst after accelerated test) / crushing strength of catalyst initially X 100

**[Table 1]**

| | Catalytic activity | Crushing strength (N) | Average particle size of coprecipitated slurry (µm) |
|---|---|---|---|
| Example 1 | 100 | 51 | 25 |
| Example 2 | 103 | 67 | 24 |
| Example 3 | 95 | 49 | 18 |
| Comparative Example 1 | 72 | 31 | 153 |
| Comparative Example 2 | 67 | 22 | 182 |
| Comparative Example 3 | 81 | 61 | 162 |
| Comparative | 89 | 29 | 15 |
| Example 4 | | | |
| Comparative Example 5 | 57 | 123 | 21 |

**[Table 2]**

| | N₂O pulse chemisorption | Accelerated test | |
|---|---|---|---|
| | Cu surface area (m²/g) | Si elution ratio (%) | Crushing strength decline ratio (%) |
| Example 1 | 7 | 0 | 0 |
| Example 2 | 9 | 0 | 0 |
| Example 3 | 5 | 0 | 0 |
| Comparative Example 1 | 6 | 11 | 20 |
| Comparative Example 2 | 6 | 12 | 14 |
| Comparative Example 3 | 8 | 14 | 17 |
| Comparative Example 4 | 2 | 37 | 49 |
| Comparative Example 5 | 2 | 26 | 35 |

As seen from Tables 1 and 2, the catalysts for preparation of neopentyl glycol of Examples 1 to 3 exhibited superior catalytic activity as compared to the catalysts of Comparative Examples 1 to 7, with a Cu surface area of 3 m²/g to 10 m²/g upon N₂O pulse chemisorption analysis and a Si elution ratio of 5 wt% or lower after the accelerated test.

For the catalyst for preparation of neopentyl glycol of Comparative Example 1, catalytic activity declined because the heat of neutralization was not controlled because of the temperature maintained during the addition of the alkaline precipitant. In addition, the catalyst for preparation of neopentyl glycol of Comparative Example 2 showed decline in catalytic activity and physical stability due to the change in the coprecipitated slurry under the high aging temperature condition. In addition, the catalyst for preparation of neopentyl glycol of Comparative Example 3 showed decline in catalytic activity and physical stability due to the change in the crystal phase of the catalyst precursor at the high drying temperature. In addition, the catalysts for preparation of neopentyl glycol of Comparative Examples 4 and 5 showed low physical stability and low catalytic activity because Si elution was not controlled effectively since the inorganic binder or organic compound was not used.

In addition, the catalyst for preparation of neopentyl glycol according to an exemplary embodiment of the present application, which is a catalyst extrusion-molded into a cylindrical shape, is applicable to a fixed-bed reactor of a commercial scale, to which an unmolded powder catalyst is difficult to be applied, and the possibility of drifting or differential pressure caused by wearing, crushing, etc. during long-term use of the catalyst may be decreased.

Accordingly, since the catalyst for preparation of neopentyl glycol according to an exemplary embodiment of the present application can maintain catalytic activity even under high-temperature, high-pressure reaction conditions due to superior strength, reactivity and stability of the catalyst, it can improve the stability of a process for preparing neopentyl glycol with superior catalytic activity.

## Claims

1. A copper silicate-based catalyst comprising copper and silica, wherein
the copper silicate-based catalyst has a crushing strength of 40 N or higher,
the copper silicate-based catalyst has a Cu surface area of 3 m²/g to 10 m²/g upon N₂O pulse chemisorption analysis, and
the copper silicate-based catalyst has a Si elution ratio of 5 wt% or lower after an accelerated test according to Method 1:
[Method 1]
after immersing the copper silicate-based catalyst in a hydroxypivaldehyde (HPA) solution and conducting reaction at 180 °C for 6 hours under hydrogen atmosphere, the elution ratio of Si is calculated according to Equation 1: [Equation 1] 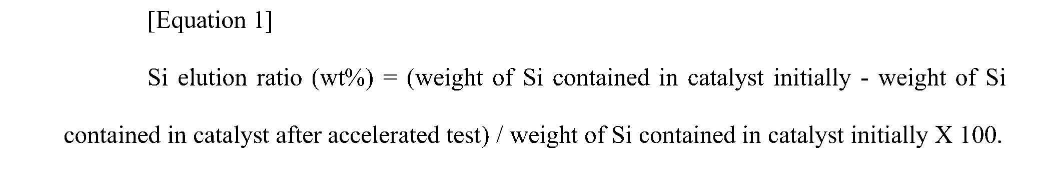 Si elution ratio (wt%) = (weight of Si contained in catalyst initially - weight of Si contained in catalyst after accelerated test) / weight of Si contained in catalyst initially X 100.

2. The copper silicate-based catalyst according to claim 1, wherein the copper silicate-based catalyst has a crushing strength decline ratio of 10% or lower after an accelerated test according to Method 2:
[Method 2]
after immersing the copper silicate-based catalyst in a hydroxypivaldehyde (HPA) solution and conducting reaction at 180 °C for 6 hours under hydrogen atmosphere, the crushing strength decline ratio is calculated according to Equation 2: [Equation 2] 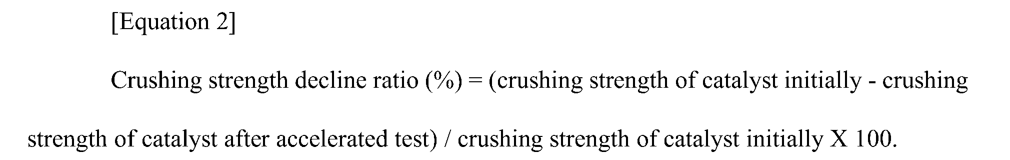 Crushing strength decline ratio (%) = (crushing strength of catalyst initially - crushing strength of catalyst after accelerated test) / crushing strength of catalyst initially X 100.

3. The copper silicate-based catalyst according to claim 1, wherein the copper silicate-based catalyst is a catalyst extrusion-molded into a cylindrical shape with a diameter of 2 mm to 6 mm and a height of 2 mm to 10 mm.

4. The copper silicate-based catalyst according to claim 1, wherein the copper silicate-based catalyst is for preparation of neopentyl glycol.

5. A method for preparing the copper silicate-based catalyst according to any of claims 1 to 4, comprising:
a step of preparing a coprecipitated slurry by adding a silica sol and an alkaline precipitant to an aqueous solution containing a copper precursor;
a step of aging the coprecipitated slurry at 80 °C to 90 °C for 3 hours or longer, and then filtering the same;
a step of preparing a dried product by drying the filtered coprecipitated slurry at 80 °C to 90 °C;
a step of mixing the dried product with an inorganic binder comprising a fiber-based binder and graphite; and an organic compound, and then preparing a catalyst precursor by molding the same; and
a step of drying and sintering the catalyst precursor,
wherein temperature is maintained at 15 °C to 35 °C during the addition of the alkaline precipitant.

6. The method for preparing the copper silicate-based catalyst according to claim 5, wherein the alkaline precipitant comprises one or more of NH₄OH, (NH₄)₂CO₃, NH₄HCO₃, CH₄N₂O, NaOH and Na₂CO₃.

7. The method for preparing the copper silicate-based catalyst according to claim 5, wherein the coprecipitated slurry has an average particle size of 10 µm to 150 µm.

8. The method for preparing the copper silicate-based catalyst according to claim 5, wherein the fiber-based binder comprises one or more of glass fiber, carbon fiber, aramid fiber, alumina fiber, aluminum silicate fiber, silicon carbide fiber and boron fiber.

9. The method for preparing the copper silicate-based catalyst according to claim 5, wherein a content of the inorganic binder is 0.1 wt% or more and less than 15 wt% based on a total weight of the dried product.

10. The method for preparing the copper silicate-based catalyst according to claim 5, wherein a content of the organic compound is 0.1 wt% to 50 wt% based on a total weight of the dried product.

11. The method for preparing the copper silicate-based catalyst according to claim 5, wherein the organic compound comprises one or more of polyvinyl alcohol, isopropyl alcohol, ethanol, polyacrylate, polyethylene glycol, glycerin, starch, dextrin, wax, methyl cellulose, carboxymethyl cellulose, hydroxypropyl methyl cellulose, paraffin, lignosulfonate, stearic acid and palmitic acid.

12. A method for preparing neopentyl glycol, comprising a step of conducting hydrogenation reaction by adding a hydroxypivaldehyde (HPA) solution and hydrogen to a hydrogenation reactor, wherein
the hydrogenation reactor comprises the copper silicate-based catalyst according to any of claims 1 to 4.
